(19) Europäisches Patentamt / European Patent Office / Office européen des brevets

(11) **EP 4 129 310 B1**

(12) **EUROPEAN PATENT SPECIFICATION**

(45) Date of publication and mention of the grant of the patent:
**22.04.2026 Bulletin 2026/17**

(21) Application number: **21779640.8**

(22) Date of filing: **29.03.2021**

(51) International Patent Classification (IPC):
$A61K\ 51/12^{(2006.01)}$    $A61P\ 35/00^{(2006.01)}$
$A61K\ 101/02^{(2006.01)}$    $A61K\ 103/10^{(2006.01)}$
$A61K\ 35/20^{(2006.01)}$    $A61K\ 47/46^{(2006.01)}$
$A61K\ 49/06^{(2006.01)}$

(52) Cooperative Patent Classification (CPC):
**A61K 51/1203; A61P 35/00**

(86) International application number:
**PCT/ES2021/070216**

(87) International publication number:
**WO 2021/198545 (07.10.2021 Gazette 2021/40)**

(54) **USE OF BREASTMILK EXOSOMES AS RADIOISOTOPE VEHICLE FOR THE DIAGNOSIS AND TREATMENT OF NEOPLASMS**

VERWENDUNG VON MUTTERMILCHEXOSOMEN ALS RADIOISOTOPENVEHIKEL ZUR DIAGNOSE UND BEHANDLUNG VON NEOPLASMEN

UTILISATIONS D'EXOSOMES DE LAIT MATERNEL COMME VÉHICULE DE RADIO-ISOTOPES POUR LE DIAGNOSTIC ET LE TRAITEMENT DE NÉOPLASIES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.04.2020 ES 202030272**

(43) Date of publication of application:
**08.02.2023 Bulletin 2023/06**

(73) Proprietors:
- **Instituto de Salud Carlos III (ISCIII)**
  **28029 Madrid (ES)**
- **Instituto Aragonés de Ciencias de la Salud (IACS)**
  **50009 Zargoza (ES)**
- **Fundación Agencia Aragonesa para la Investigación**
  **y el Desarrollo (ARAID)**
  **50018 Zaragoza (ES)**
- **Fundacion Universidad Francisco de Vitoria (UFV)**
  **28223 Pozuelo de Alarcón - Madrid (ES)**

(72) Inventors:
- **DE LA VIEJA ESCOLAR, Antonio**
  **28029 Madrid (ES)**
- **MARTÍN DUQUE, Mª Pilar**
  **50018 Zaragoza (ES)**
- **CRESPO BARREDA, Alejandra**
  **28223 Pozuelo de Alarcón - Madrid (ES)**

(74) Representative: **Pons IP**
**Glorieta Rubén Darío 4**
**28010 Madrid (ES)**

(56) References cited:
**US-A1- 2018 325 998**

- **CRESPO-BARREDA ALEJANDRA: "Uso del transportador de NA/I (NIS) end�geno vehiculizado por c�lulas troncales y exosomas para terapia y diagn�stico del c�ncer", 1 November 2020 (2020-11-01), XP093204889, Retrieved from the Internet <URL:https://ddfv.ufv.es/entities/publication/09d3497c-d436-499c-a4e7-1777dc4ecdf8> [retrieved on 20240913]**

**(Cont. next page)**

- SON SEUNG HYUN ET AL: "A novel strategy of transferring NIS protein to cells using extracellular vesicles leads to increase in iodine uptake and cytotoxicity", INTERNATIONAL JOURNAL OF NANOMEDICINE, 7 March 2019 (2019-03-07), New Zealand, pages 1779 - 1787, XP093204592, ISSN: 1178-2013, Retrieved from the Internet <URL:https://www.dovepress.com/article/download/44481> [retrieved on 20240913], DOI: 10.2147/IJN.S189738
- AQIL FARRUKH ET AL: "Milk exosomes - Natural nanoparticles for siRNA delivery", CANCER LETTERS, vol. 449, 1 May 2019 (2019-05-01), pages 186 - 195, XP085622167, ISSN: 0304-3835, DOI: 10.1016/J.CANLET.2019.02.011
- CRESPO-BARREDA A ET AL.: "Use of Placental MSCs and their exosomes as theragnostic agents for cancer treatment and diagnostic", HUMAN GENE THERAPY, vol. 30, no. 11, 11 January 2019 (2019-01-11), pages A53, XP055927498, ISSN: 1557-7422, DOI: 10.1089/hum.2019.29095.abstracts
- A CRESPO-BARREDA; E HERRERO; M IGLESIAS; M QUINTANILLA; A DE LA VIEJA; P MARTIN- DUQUE: "P149: Placental MSCs as vehicles for the endogenous Na/I symporter (hNIS): a new theragnostic strategy", HUMAN GENE THERAPY, vol. 27, no. 11, 1 November 2016 (2016-11-01) - 21 October 2016 (2016-10-21), GB
, pages 1 - 2, XP009536297, ISSN: 1043-0342, DOI: 10.1089/hum.2016.29035.abstracts
- AGRAWAL ASHISH K ET AL.: "Milk-derived exosomes for oral delivery of paclitaxel", NANOMEDICINE-NANOTECHNOLOGY BIOLOGY AND MEDICINE, vol. 13, no. 5, 30 June 2017 (2017-06-30), pages 1627 - 1636, XP055541721, ISSN: 1549-9634, [retrieved on 20170700], DOI: 10.1016/j.nano. 2017.03.00 1
- MUNAGALA RADHA ET AL.: "Bovine milk-derived exosomes for drug delivery", CANCER LETTERS, vol. 371, no. 1, 30 November 2015 (2015-11-30), pages 48 - 61, XP055509175, ISSN: 0304-3835, [retrieved on 20160000], DOI: 10.1016/j.canlet. 2015.10.02 0
- DE LA VIEJA ANTONIO ET AL.: "Role of iodide metabolism in physiology and cancer", ENDOCRINE-RELATED CANCER, vol. 25, no. 4, 31 March 2018 (2018-03-31), pages R225 - R245, XP055927568, ISSN: 1351-0088, DOI: 10.1530/ERC-17-0515
- TAZEBAY UYGAR H ET AL.: "The mammary gland iodide transporter is expressed during lactation and in breast cancer", NATURE MEDICINE, vol. 6, no. 8, 31 July 2000 (2000-07-31), pages 871 - 878, XP002944242, ISSN: 1078-8956, [retrieved on 20000800], DOI: 10.1038/78630
- GARDEAZABAL L., YNDRIAGO L., CRESPO-BARREDA A., MARTIN-DUQUE P., ALDAZABAL J., PAREDES J., IZETA A.: "Topical use of exosomes from human maternal milk accelerates acute cutaneous wound healing", JOURNAL OF INVESTIGATIVE DERMATOLOGY, vol. 139, no. 9, 31 August 2019 (2019-08-31), pages 327, XP055927589, ISSN: 0022-202X
- GONZÁLEZ MARÍA ISABEL, MARTÍN-DUQUE PILAR, DESCO MANUEL, SALINAS BEATRIZ: "Radioactive Labeling of Milk-Derived Exosomes with <99m>Tc and In Vivo Tracking by SPECT Imaging", NANOMATERIALS, vol. 10, no. 6, 30 May 2020 (2020-05-30), pages 1062, XP055927593, ISSN: 2079-4991, DOI: 10.3390/nanol0061062

Remarks:
   The complete document including Reference Table(s) and the Sequence Listing(s) can be downloaded from the EPO website

## Description

The present invention falls within the field of medicine, specifically within the theragnostic methods (diagnosis and therapy) of tumours that use vehicles capable of specifically transporting either drugs, contrast agents or visualization agents, preferably radioactive isotopes, to the neoplastic areas.

## BACKGROUND OF THE INVENTION

The main problem for the application of antitumor therapies often lies in the difficulty of efficiently transporting therapeutic agents to pathological targets. Among the vehicles proposed to overcome this limitation, the most popular are mammalian cells and, among them, stem cells. Unlike other cellular vehicles, mesenchymal stem cells or MSCs (from the English term "mesenchymal stem cells") have certain characteristics that make them superior, such as their capacity for self-renewal, the potential to differentiate themselves into different types of cells and their ability to migrate and integrate into inflamed or damaged areas, such as tumors. Although the complete mechanism that mediates this phenomenon is still unclear, it is known that the release of cytokines in the tumor stroma and their interaction with specific surface receptors, present in MSCs plays an important role in the migration process of these cells. The use of MSCs as vehicles of various therapies is currently being developed, and there are clinical trials proposing the use of patient-derived MSCs as viral vectors in the treatment of multiple types of cancer.

Recent results show that the therapeutic action of stem cells might be due to paracrine signaling and these paracrine effects are thought to be mainly mediated by extracellular vesicles (EVs), including exosomes, which can transfer bioactive proteins and genetic material to target cells.

EVs, including exosomes, are micro- or nano-sized vesicles derivatives of cells and that transport nucleic acids, proteins, lipids and other molecules. EVs transfer material to cells with which they fuse and play an important role in cell-to-cell communication. These vesicles are secreted by a variety of cell types and have recently emerged as promising biomarkers for a number of human diseases, including cancer and neurodegeneration, as well as drug delivery systems.

Moreover, the iodine and sodium (Na+/I-) transporter or symporter gene (NIS) is expressed in the thyroid, where it mediates the entry and accumulation of iodine from the diet for the production of thyroid hormones T3 and T4. It also mediates the flow of iodine to other tissues such as the intestine, stomach, and salivary glands to carry iodine to the blood and later to the thyroid. NIS is also expressed in the placenta during pregnancy and in the lactating mammary gland, in both cases to supply iodine from the mother to the fetus or baby. Thus, it has been proposed that NIS, as a carrier protein, can be used to concentrate radioisotopes inside cells. Therefore, the combination of NIS and the use of radioisotopes can be used as reagents in visualization techniques, such as SPECT or PET with radioisotopes such as $^{123}$I, $^{124}$I, $^{125}$I or $^{99m}$TcO4-, and/or as a therapeutic agent in radiotherapy to eliminate tumor cells with isotopes such as $^{131}$I, $^{188}$ReO4- or $^{211}$At *(De la Vieja Antonio, Santisteban Pilar, 2018, Endocrine-Related Cancer, Vol. 25, No. 4, R225-R245)*.

For decades, $^{131}$I-radioiodine therapy has been the essential adjuvant therapy for the treatment of thyroid cancer, with considerable success and relatively minor side effects compared to other therapies. The ability of NIS to accumulate $^{131}$I, is the basis of this therapy and one of the preferred molecular options in theragnosis (diagnosis+ therapy). In recent decades, NIS-translocated radioisotopes represent one of the major advances in therapeutic nuclear oncology *(Chung, JK, GJ Cheon, 2014, Endocrinol Metab, 29(3): 233-239)*. NIS has a unique role in this process, as it acts as a reporter or therapeutic modality. That is, NIS can be used as a reporter for non-invasive imaging techniques such as PET and SPECT, with the addition that it has some advantages over other reporter genes used by these techniques, and also has better resolution and penetration than those used by other non-nuclear techniques, such as bioluminescence and fluorescence.

However, one of the problems that remains to be solved is how to direct this gene specifically to the tumoral area.

Human mesenchymal stem cells (hMSCs) from the placenta and their exosomes are known to endogenously express NIS. In addition, exosomes from placental MSCs have been shown to have a tropism for tumors, as other MSCs do. As a consequence, they can serve as radioaccumulative vehicles, characteristic that allows them to be used in visualization techniques or for radiotherapy, if cancer is treated with $^{131}$I or with any other theragnostic radioisotope accumulated by NIS *(Crespo-Barreda A., et al., 2019, Journal of Extracellular Vesicles, Vol 8, No. Supplement 1, 105 - 106; Crespo-Barreda A., et al., 2016, Human Gene Therapy, Vol. 27, No. 11, page A91; Crespo-Barreda A., et al., 2019, Human Gene Therapy, vol. 30, no. 11, page A53)*.

Exosomes and EVs from various sources are being used to reach the tumor site. Agrawal Ashish K. *et al. (Agrawal Ashish K., et al., 2017, Nanomedicine, Vol. 13, No. 5, 1627-1636)* described the advantages of oral administration of bovine milk's exosomes as vehicles to transport paclitaxel to tumor with a high rate of tumoral growth inhibition (60%) and lower systemic and immunological toxicity compared to conventional administration (intravenous) of paclitaxel, so that although its arrival was not visualized, it was assumed that the exosomes reached the tumor, exerting their antitumoral effect.

The versatility of milk exosomes as vehicles for antitumoral substances and their ability to reach tumor cells has also been described in Munagala Radha, et al.,

2016, Cancer Letters, Vol. 371, No. 1, 48-61.

Finally, in Tazebay Uygar H., et al., 2000, Nature Medicine, Vol. 6, N° 8, 871-878, the authors showed that the sodium-iodine co-transporter hNIS mediates iodine transport in healthy infants, but not in non-infants, as it is present in the mammary gland as well as in mammary tumors. They also show that *in vivo* breast adenocarcinomas from transgenic mice accumulate iodine due to NIS and that more than 80% of human breast cancer samples express hNIS. Results of their work indicate that hNIS is expressed in the lactating mammary gland and in certain circumstances in breast cancer, and that radioiodine could be accumulated in those tumors.

In summary, besides the side effects, the main problem for the application of antitumor therapies lies in the difficulty of the effective transportation of the therapeutic agents to the pathological targets. This highlights a clear need to develop new efficient and easy-to-follow transport vehicles that allow neoplastic regions to be visualized and ensure that therapies reach tumors specifically. Therefore, there is a need to develop new vehiculization strategies, more effective and safer, capable of specifically and homogeneously reach the entire tumor mass, including the central hypoxic regions, in order to improve theragnosis in the tumors.

## DESCRIPTION OF THE INVENTION

The inventors of the present invention have shown that exosomes and other extracellular vesicles (EVs) derived from breast milk have tropism towards tumors and moreover express high levels of endogenous NIS, even higher than those shown by exosomes and other EVs from mesenchymal stem cells (MSCs) from placenta. This allows the accumulation of radioisotopes in their interior, preferably radioiodide isotopes (but also any other theragnostic radioisotope that could be accumulated by NIS), thus enabling its use as a vehicle or collector/accumulator of radioactive isotopes, specifically towards the neoplastic areas for their visualization and /or treatment.

Thus, the present invention proposes exosomes and other EVs from milk (both human and from other species) together with radioactive isotopes (i) for use to visualize tumor nodules and thus serve for the diagnosis and monitoring of tumors, preferably solid tumor masses and the fate of these EVs, and (ii) for use in antitumoral therapy, since through the accumulation of radioisotopes accumulative by NIS (for example, $^{131}$I) in tumors, tumor nodules are eliminated in an specific way, with the additional advantage that side effects hardly appear due to its high tumor selectivity.

In addition, the inventors also showed that milk exosomes and other milk EVs also migrate towards metastases, so their combination with radioisotopes would also serve to diagnose, monitor and treat tumor metastases.

Therefore, **in the** present **invention it is shown that** milk exosomes and other milk EVs are optimal vehicles for transporting and accumulating radioisotopes, specifically in tumors, due to their very high tropism towards tumor areas, while they are not detected in other regions. Thanks to their endogenous expression of the **NIS** gene, they accumulate radioisotopes (mainly iodinated), so that it is possible (i) to visualize these exosomes in tumor masses by techniques of imaging such as PET or SPECT, serving this for the diagnosis of neoplasms and/or for monitoring therapies, and (ii) to eliminate tumors by the accumulation of therapy with radioisotopes accumulated by NIS (such as $^{131}$I). In fact, the examples shown below show, among other things, that this accumulation of transportable and accumulative radioisotopes by NIS gives rise to a very significant inhibition of tumor growth.

The possibility of carrying out the diagnosis and monitoring or therapy of tumors, using the approach proposed here, will depend on the type of radioisotope that will be used together with the exosome or vesicle. Thus, for example, $^{123}$I, $^{124}$I, $^{125}$I or $^{99m}$TcO4- can be used for PET and/or SPECT visualization, while $^{131}$I, $^{188}$ReO4- or $^{211}$At can be used for therapy, although any other NIS-accumulative radioisotope could also be used in the context of the present invention.

The advantages of the object of the invention, compared to the use of MSCs or their exosomes as vehicles, are that cells are not used here and that the EVs used here do not come from stem cells, thus avoiding ethical conflicts and possible harmful effects (teratogenesis, etc.). In addition, the expression of endogenous NIS in milk exosomes and other milk EVs is higher, and therefore the theragnostic effect is better, than in MSCs exosomes. It should also be noted that in the present invention sensitivity reaches even metastases. A greater amount of exosomes and other vesicles is obtained from milk than from the placental MSCs and its method of obtention is less invasive. Finally, the present invention supposes a cheap, reproducible and simple method that is common to several species and that is easy to implement in the clinic.

For all those reasons, one aspect of the invention refers to a composition, hereinafter the "composition of the invention", that comprises:

(I) Extracellular vesicles (EVs), preferably exosomes, secreted, and subsequently isolated, by cells expressing the protein NIS, wherein said EVs, preferably exosomes, express the NIS protein, and
(II) at least one radioactive isotope or radioisotope, preferably but not exclusively, a radioiodide isotope,

wherein the extracellular vesicles, preferably exosomes, secreted by cells expressing NIS protein have been isolated from milk, more preferably milk from breastfeeding.

In a preferred embodiment of the composition of the invention, it comprises the two elements: (i) and (ii) separately. In another preferred embodiment of the composition of the invention, the EVs, preferably the exosomes, indicated in (i) have been injected with the radioactive isotope indicated in (ii), so that they comprise it internally.

The "radioactive isotope" to which the present invention refers, can be any radioisotope that can be transported and accumulated by NIS. Preferably, the radioactive isotope is selected from among the most common radioisotopes that can be transported and accumulated by NIS. In a more preferred embodiment of the composition of the invention, the radioactive isotope is selected from the list consisting of: $^{131}I$, $^{123}I$, $^{124}I$, $^{125}I$, $^{211}At$, $^{99m}TcO4-$, $^{188}ReO4-$, and $^{18}F-BF4-$. In a more preferred embodiment, the radioactive isotope is (for therapy) $^{131}I$ or (for imaging) $^{99m}TcO4-$, even more preferably $^{131}I$. The radioactive isotope $^{131}I$ is also called RAI or $I^{-131}$, or radioiodine.

The "NIS protein" ("Sodium/iodide symporter") or "iodine and sodium co-transporter/symporter" mediates the absorption of iodide in follicular cells of the thyroid for the biosynthesis of thyroid hormones (T3 and T4). This protein is also expressed in other tissues such as the gastric mucosa, the salivary glands or the digestive tract to capture circulating iodine from the diet. It is encoded by the gene called *SLC5A5* (for Solute Carrier family 5 member 5). There are 254 orthologs of human NIS in vertebrates, 130 of which are found in mammals. All of those orthologs potentially have the ability to transport sodium-dependent iodide, as well as any of the radioisotopes that are carried by the human NIS protein. In the case of human species, various types of primates, rat, mouse, pig, whale, frog, and zebrafish, this is experimentally proven. The regions of the protein involved in iodide transport are fully conserved in all the orthologous proteins of human NIS. Therefore, the NIS protein to which the present invention refers, includes the human NIS protein and any of its orthologs in other species, preferably in primates (such as, for example, but not limited to, chimpanzee or macaque), rat, mouse, pig, dog, cow, hamster, whale, frog and fish zebra.

Preferably, the NIS protein referred to in the present invention comprises, more preferably consists of, an amino acid sequence that has at least 55%, 80%, 85%, 89%, 90%, 91%, 92%, 93%, 94 %, 95%, 96%, 97%, 98%, 99% or 100% of identity with SEQ ID NO: 1 (human NIS protein or hNIS) and retains the amino acids responsible for iodide transport.

In another preferred embodiment of the composition of the invention, the NIS protein is human, more preferably the human NIS protein (hNIS) comprises, preferably consists of, SEQ ID NO: 1.

NIS is a plasma membrane protein, so EVs, preferably exosomes, from NIS-expressing cells will also comprise (express) NIS. The methods to detect whether said EVs, preferably exosomes, express the NIS protein are those commonly known in the art, such as western-blot, immunofluorescence techniques or ELISA-type enzymatic assays, among others.

The "extracellular vesicles" or "EVs" to which the present invention refers, are microvesicles or nanovesicles, preferably nanovesicles, more preferably exosomes, secreted by cells. The EVs are heterogeneous and their content is very varied, from RNA, to lipids, carbohydrates and proteins, including both nuclear DNA and mitochondrial. In general, these vesicles contain a large assortment of proteins, such as enzymes, cytoskeletal elements, transcription factors, integral membrane associated proteins, histocompatibility complexes, etc. They also contain different types of RNA, such as messengers, interfering RNAs and small non-coding RNAs, some of these mRNAs have even been shown that can be translated into proteins in target cells. Lipids are part of their membranes, which may contain phosphatidyl serine, lysophosphatidylcholine, sphingomyelin, and acylcarnitines. These vesicles are enriched in cholesterol and sphingolipids. The release of these vesicles by cells can be spontaneous or induced.

"Exosomes" are solid extracellular nanovesicles that are actively released by cells into the extracellular matrix and that contain mixtures of proteins, nucleic acids, and other biomolecules such as lipids and/or miRNAs. Exosomes are released into the extracellular space after fusion of the multivesicular bodies with the plasma membrane. This mechanism was described, and the term "exosome" coined, in the 80s of the 20th century. Preferably, the exosomes referred to in the present invention have a size between 60 and 250 nm, more preferably between 100 and 185 nm, and have a rounded shape.

The milk exosomes of the present invention express the NIS protein, preferably the human NIS protein (hNIS), and more preferably they also express at least the exosome markers ALIX, CD9 and CD63.

Milk exosomes can be isolated by any of the methods known in the state of art for this purpose, for example, but not limited to, those described in: Drug Dev Ind Pharm. 2019 Mar;45(3): 359-364.doi:10.1080/03639045.2018.1539743*; Reprint Biol. 2017 Dec;17(4):341-348. doi: 10.1016/j.repbio.2017.09.007.; or* J. Vet. Med. Sci. 74(11): 1523-1525, 2012 doi: 10.1292/jvms.12-0032. For example, but without limitation, these exosomes are isolated by a first step eliminating the fat from breast milk, after an optional step of freezing / thawing of the milk, a second step in which the product resulting from the first step is incubated with rennet, for preferably 15 to 20 min, a third centrifugation step in which proteolysis products such as casein are removed, and a fourth step comprising several, preferably two, ultracentrifugations. Finally, the resulting product can be washed once or several times, preferably twice.

EVs, preferably exosomes, isolated from milk have been separated by procedures well known in the state of art, from other milk components, such as lipids, cells or debris.

Among the advantages of the clinical use of EVs, preferably exosomes, it can be highlighted that their use would avoid the use of cells to treat the patients, which may have mutated or damaged DNA. Secondly, and for systemic applications, as could be the case in therapeutic treatments, EVs are small and circulate easily throughout the body, while in cell therapy they can remain retained since they are too large to circulate through the capillaries and their passage through some organs, such as the lungs, and could cause serious damage, such as an embolism. Third, the administered dose of EVs can circulate in the body to a greater extent and more easily compared to cells.

The "milk" to which the present invention refers can be breast milk, that is, milk from a mammal female that is in the lactating period, or milk from a mammal female that is not in the lactating period of lactation but in which the breast is being stimulated for the production of milk and, therefore, for the expression of NIS. Preferably, the milk referred to in the present invention is breast milk. The origin of said milk can be from any mammal female, for example, but not limited to, the following species: human, cow, goat, sheep, dromedary, camel, dog, cat, pig, rat, etc. In a more preferred embodiment, the milk is breast milk from a lactating mammal female. In a more preferred embodiment, the milk is breast milk from a lactating human female.

The elements comprised in the composition of the invention can be administered simultaneously or sequentially, or, as explained above, the Evs, preferably the exosomes, comprised in said composition can be loaded with the radioisotope, ie the radioisotope may have been incorporated into the EV, preferably into the exosome.

In another preferred embodiment, the composition of the invention is a pharmaceutical composition preferably formulated for intravenous or intraperitoneal administration.

Another aspect refers to the EVs, preferably exosomes, described in the present invention for use as vehicles for the transport and/or *in vivo* accumulation of radioactive isotopes towards neoplastic areas in a subject.

Another aspect of the invention refers to the composition of the invention for its use as a medicine, wherein said medicine can be for human or animal use. Said medicament may be intended for the treatment, prevention, diagnosis and/or monitoring of neoplasms in a subject or the monitoring and visualization of the vehicle (radioisotope) within the body of the subject.

Another aspect of the invention refers to the composition of the invention for its use in the treatment and/or prevention of neoplasms. In a preferred embodiment of this aspect of the invention, the composition comprises the radioactive isotope $^{131}$I, $^{188}$ReO4- and/or $^{211}$At, more preferably $^{131}$I.

The term "neoplasms" used in the present invention includes both benign and malignant tumors (cancer), as well as metastases. Likewise, within malignant tumors, and within the scope of the present invention, are include, but are not limited to, both solid tumors and hematological tumors.

In another preferred embodiment, the neoplasm referred to in the present invention is a solid or hematological tumor. More preferably, the solid tumor is a cancer. Even more preferably, said cancer is selected from the list consisting of: melanoma, multiple myeloma, leukemia, lymphoma, ovarian cancer, cervical cancer, lung cancer, liver cancer, breast cancer, pancreatic cancer, gastric cancer, colorectal cancer, head and neck cancer, mouth cancer, stomach cancer, brain tumors, and thyroid cancer. In a particular embodiment, the cancer is selected from the list consisting of: melanoma, ovarian adenocarcinoma, cervical cancer, and thyroid cancer.

Another aspect of the invention refers to the composition of the invention for use in the diagnosis and/or *in vivo* monitoring of neoplasms.

Another aspect of the invention refers to the use of the composition of the invention as an imaging or contrast reagent or agent for visualization of neoplasms in a subject.

The term "diagnosis" refers to the process of identifying the presence or absence of neoplasms in a subject. The term "monitoring" refers to the process of identifying events that occur during the course of neoplasms, and includes, but is not limited to, the determination of the presence or absence of metastases, the determination of the response to a therapeutic treatment, as well as the determination of the progression (worsening) or regression (improvement) of the neoplasms.

In a preferred embodiment of this aspect of the invention, the visualization of neoplasms is performed using the PET or SPECT technique.

"PET" is the technique known in the state of the art as positron emission tomography.

"SPECT" is the technique known in the state of the art as single photon emission computed tomography. Since they are well known techniques, the person skilled in the art will know how to apply them according to what is stated in the present invention.

In another preferred embodiment of this aspect of the invention, the composition of the invention comprises a radioactive isotope that is selected from the list consisting of: $^{123}$I, $^{124}$I, $^{125}$I, $^{211}$At, $^{99m}$TcO4-, $^{188}$ReO4-, and $^{18}$F-BF4-, preferably $^{124}$I and $^{99m}$TcO4-.

If the chosen visualization technique is PET, the composition of the invention preferably comprises a radioactive isotope that is selected from the list consisting of: $^{124}$I, $^{211}$At, $^{18}$F-BF4-.

If the chosen visualization technique is SPECT, the composition of the invention preferably comprises a radioactive isotope that is selected from the list consisting of: $^{123}$I, $^{125}$I, $^{131}$I, $^{99m}$TcO$_4$- and $^{188}$ReO$_4$-.

In another preferred embodiment, the neoplasm is a solid or hematological tumor, more preferably a cancer, even more preferably a cancer selected from the list consisting of: melanoma, multiple myeloma, leukemia, lymphoma, ovarian cancer, cervical cancer, lung cancer, liver cancer, breast cancer, pancreatic cancer, gastric cancer, colorectal cancer, head and neck cancer, mouth cancer, stomach cancer, brain tumor and thyroid cancer. In a particular embodiment, the cancer is selected from the list consisting of: melanoma, ovarian adenocarcinoma, cervical cancer and thyroid cancer.

Another aspect of the invention relates to a method of *in vivo* visualizing neoplasms in a subject wherein the mentioned method comprises the use of the composition of the invention. Preferably, the mentioned method comprises the following steps: (a) administering to the subject, preferably by injection, the EVs, preferably the exosomes, described in the present invention, (b) administering to the subject, simultaneously or sequentially to the administration of step (a), at least one radioactive isotope, unless it has been previously incorporated into the EVs, in which case a single administration will be made, in stage (a), of the EVs loaded with the radioactive isotope, and (c) visualizing the destination of the EVs to the neoplasm, thus examining the presence of neoplasms in the subject by identifying the location of the radioactive isotope administered in step (a or b). Step (c) of this method can be performed using the visualization techniques described earlier in this description.

Another aspect of the invention relates to the composition of the invention for use in a method for the treatment and/or prevention of neoplasms in a subject, wherein the mentioned method comprises the following steps: (a) administering to the subject, preferably by injection, the EVs, preferably the exosomes, described in the present invention, and (b) administering to the subject, simultaneously or sequentially to the administration of step (a), at least one radioactive isotope. Alternatively, the mentioned method comprises the step of administering to the subject, preferably by intravenous or intraperitoneal injection, the EVs, preferably the exosomes, described in the present invention, wherein the said EVs have been loaded previously with a radioactive isotope. In a preferred embodiment of this method, the radioactive isotope is selected from the list consisting of: $^{131}$I, $^{188}$ReO4- and/or $^{211}$At, more preferably $^{131}$I.

In another preferred embodiment of these methods, the neoplasm is a solid or hematological tumor. More preferably, the solid tumor is a cancer. Even more preferably, a cancer selected from the list consisting of: melanoma, multiple myeloma, leukemia, lymphoma, ovarian cancer, cervical cancer, lung cancer, liver cancer, breast cancer, pancreatic cancer, gastric cancer, colorectal cancer, head and neck cancer, mouth cancer, stomach cancer, brain tumors and thyroid cancer. In a particular embodiment, the cancer is selected from the list consisting of: melanoma, ovarian adenocarcinoma, cervical cancer and thyroid cancer.

The term "subject", as used in the present invention, refers to a human or non-human individual, for example mammals such as dogs, cats, horses, rabbits, mice, cows, pigs, sheeps, guinea pigs, etc. Preferably, the subject to which the present invention relates is human.

Throughout the description and claims the word "comprise" and its variants are not intended to exclude other technical characteristics, additives, components or steps. Other objects, advantages and features of the invention will be apparent to those skilled in the art in part from the description and in part from the practice of the invention. The following examples and figures are provided by way of illustration, and are not intended to be limiting of the present invention.

## DESCRIPTION OF THE FIGURES

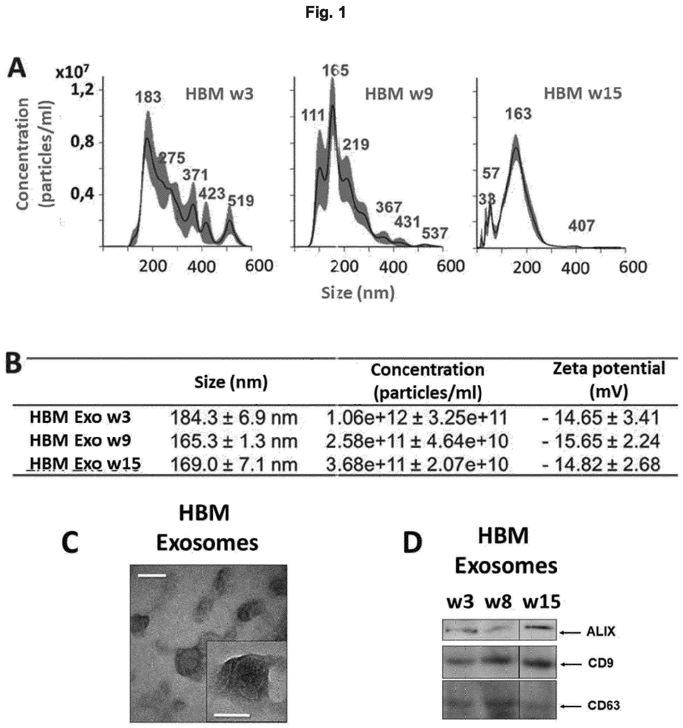

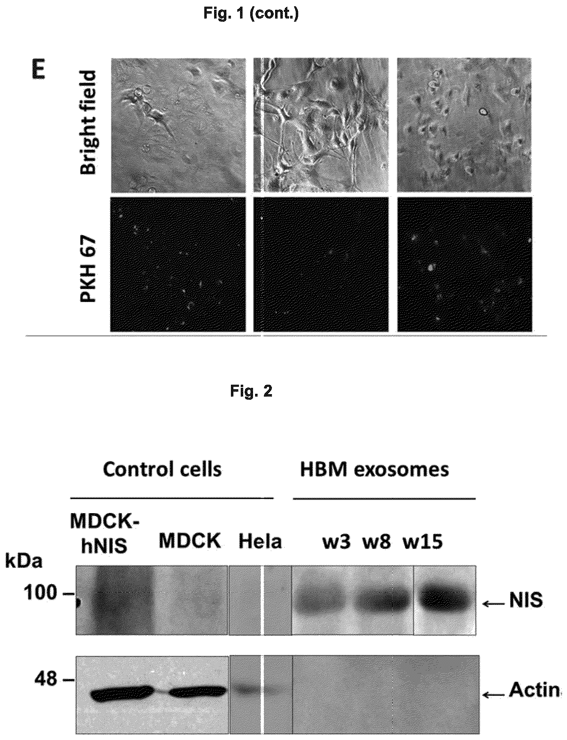

**Fig. 1. Characterization of exosomes derived from human breast milk** (HBM). Isolated exosomes from human breast milk obtained during the 3rd, 9th and 15th weeks of lactation. A) Graphical representation of the size distribution by nanoparticle tracking analysis (NTA). B) Table of size and concentration distribution obtained by NTA and zeta potential measurements by dynamic light scattering (DLS). C) Evaluation of exosome morphology by transmission electron microscopy (TEM), representative images, 50 nm scale bar. D) Representative western blot of exosome markers, Alix, CD9 and CD63). E) Representative fluorescence images of HBM exosome uptake in different cancer cell types (from left to right HeLa, B16F10, and 8505C). Exosomes were labeled with a PKH67 probe and their internalization was visualized using a fluorescence microscope (Leica DM4B).

Fig. 2. Characterization of **hNIS expression in human breast milk exosomes.** Determination of hNIS protein expression by western-blot (upper panel) in exosomes isolated from breast milk collected at different weeks during the lactation period. 50 μg of exosomes were loaded into each well. The expression of hNIS protein from exosomes was compared to positive control cells expressing NIS (MDCK-hNIS) and negative control cells not expressing NIS (MDCK and HeLa). 20 μg of control cells were loaded. Actin protein expression levels are shown (lower panel) as a loading control for samples.

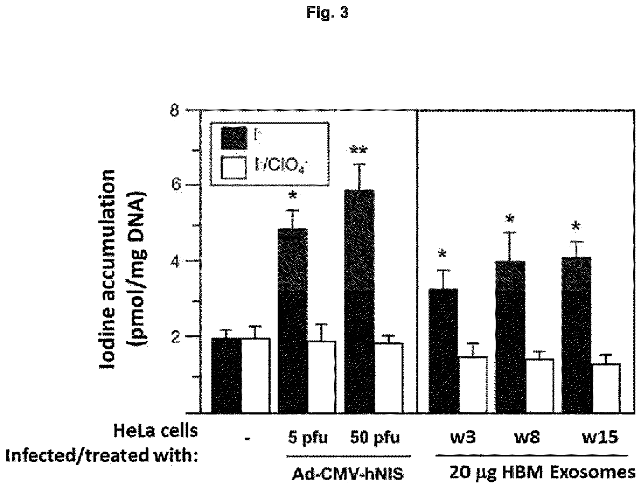

**Fig. 3. Characterization of the functional expression of hNIS** in **breast milk exosomes.** HeLa cell iodine uptake assay ($1 \times 10^5$) incubated for 45 minutes with 20 μg of exosomes isolated from human breast milk. After 48 hours of incubation, the uptake/transport of iodide into the cell was analyzed. The iodide transport assay was performed in the presence of 20 μM iodide (black bars) or 20 μM iodide plus 80 μM perchlorate (open bars). Perchlorate is a competitive inhibitor of iodide transport by NIS. HeLa cells were used as negative control and HeLa cells infected with 5 and 50 pfu of Ad-CMV-hNIS adenovirus as positive control. Uptake/transport is expressed as picomoles and normalized for the amount of cellular DNA in each well. Data represent the mean ± SE of at minus three experiments performed in triplicate.

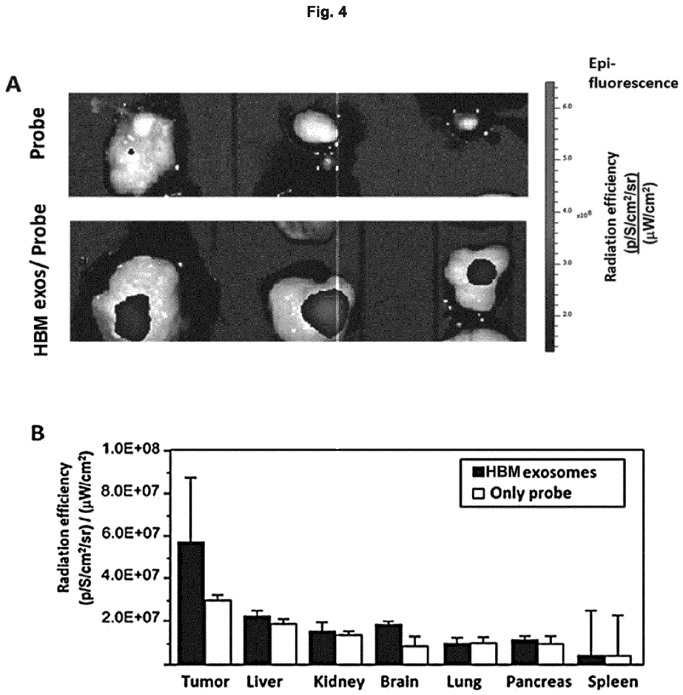

**Fig. 4. Fluorescence Imaging (FRI) biodistribution analysis of probe-labeled breast milk exosomes** in **animal tumor models.** Immunosuppressed mice with subcutaneous tumors produced by injection of $3x10^6$ HeLa tumor cells. When the tumors reached a size of approximately $0.5\,cm^2$, they were injected intravenously with 20 μg of exosomes isolated from human breast milk (HBM) previously labeled with the PKH67 probe. Using the IVIS Illumina II system, fluorescent signal was observed in tumors *in vivo.* The mice were sacrificed, different tissues and the tumor were extracted and the signal emitted by Probe into each tissue was detected with an IVIS Illumina II. Representative images of tumors from animals injected with the PKH67 probe (upper A), or with the probe-labeled HBM exosomes (lower A). Finally, the signal was quantified with the Xenogen-IVIS Software (B). Data represent the mean ±SD of at least three tumor or tissue samples.

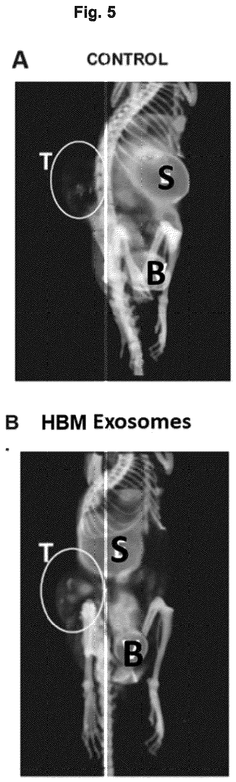

**Fig. 5. Analysis of the biodistribution and functional expression of NIS *in vivo* by SPECT-CT.** Immunosuppressed mice with subcutaneous tumors produced by injection of $3x10^6$ cells HeLa tumor cells were injected intravenously with PBS (A), or 20 μg of HBM exosomes (B). After 10 days the animals were injected intraperitoneally with a dose of 18.5 MBq of $^{99m}TcO4-$ (pertechnetate) and images were recorded by nano-SPECT-CT. The white circle indicates the position of the tumor (T). The position of the stomach (S), and bladder (B) are also indicated.

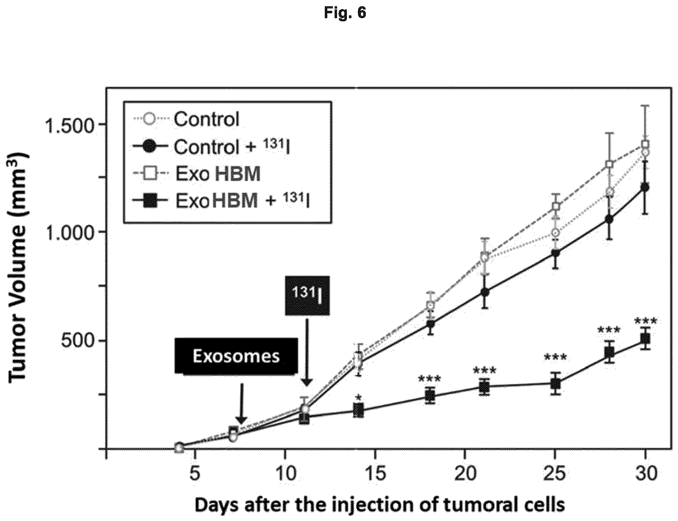

**Fig. 6. Therapeutic potential of human breast milk exosomes.** In an in vivo model, immunosuppressed mice were used in which $3x10^6$ HeLa tumor cells were implanted subcutaneously. When the tumor reached the approximate size of $100\,mm^3$, at 7 days, they were randomly separated into 2 groups. In one group PBS was injected intravenously (control animals represented with circles), and in the second group 20 μg of exosomes isolated from human breast milk (Exo HBM) (represented with squares). On day 11 after implantation of tumor cells, each group was divided into 2 subgroups randomly, intraperitoneally injecting 200 μl of PBS to the untreated subgroups (symbols with white background) or 2

mCi of $^{131}I$ to the treated subgroups (symbols with black background). Subsequently, the growth of the tumors was monitored until day 30 post-implantation of the tumor cells. 6 animals per subgroup were used. Values represent mean ± standard error. Statistical analysis was performed with a "two-sided Student's t-test" and is indicated as *p < 0.05; **p < 0.01; *** p < 0.001. The results of mice injected with exosomes and treated, versus those injected with exosomes but not treated, are indicated.

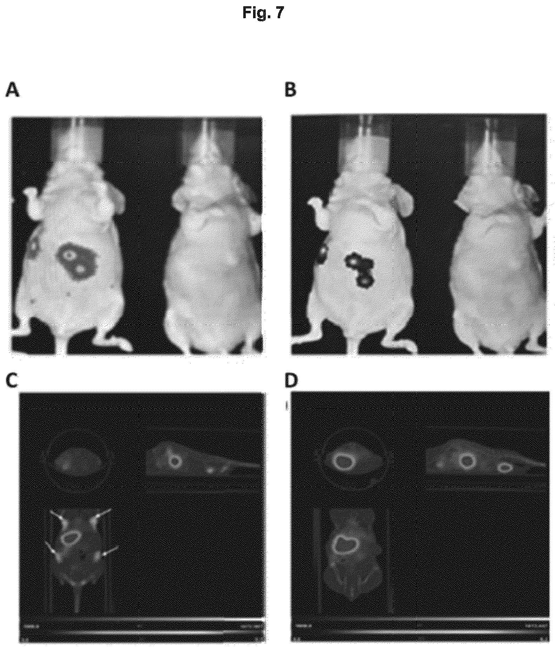

**Fig. 7. Therapeutic potential of human breast milk exosomes for diagnosis and treatment of metastases.** As an *in vivo* model, immunosuppressed mice were used, which were implanted intraperitoneally (IP) $1x10^6$ SKOV3-LUC tumor cells. When the IVIS-Illumina equipment observed more than 2 tumor nodules, 20 μg of HBM exosomes were injected intraperitoneally (IP). A) Bioluminescence images of animals with tumors and injected IP with exosomes from HBM (left) or controls (right). B) Fluorescence images of tumor-bearing animals injected IP with fluorescently probe-labeled HBM exosomes (left) or controls (right). C) Images of SPECT-CT of mice implanted intraperitoneally with $1x10^6$ SKOV3-LUC tumor cells and subsequently with 20 μg of HBM exosomes. After 10 days, the animals were injected intraperitoneally with a dose of 18.5 MBq of $^{99m}TcO4-$ (pertechnetate) and images were recorded using nano-SPECT/CT. D) SPECT-CT images of control mice.

**EXAMPLES**

Next, the invention will be illustrated by means of some assays carried out by the inventors, which show the effectiveness of the exosomes isolated from breast milk that express the NIS protein to transport radioisotopes specifically to neoplastic areas in the body for subsequent visualization in diagnosis or for therapeutic treatment.

**Example 1. Characterization of human breast milk exosomes or** human **milk exosomes (HBM).**

Human breast milk exosomes were obtained and isolated (see Example 9, Materials and methods) and proceeded to its characterization. To determine the most appropriate conditions for obtaining exosomes, as well as the subsequent functional validity of the mentioned exosomes, the exosomes from human breast milk (HBM) were characterized at the 3, 9 and 15 weeks of lactation. Using the NTA system, it was demonstrated that exosomes have a size distribution range somewhat higher than that observed in exosomes of hPMSCs (human placental MSCs), and that it goes mainly between 100 and 250 nm, with an average size of between 165 at 185 nm, and with a maximum peak of main concentration of exosomes between 160 to 185 nm (Fig. 1A). The concentration of exosomes was higher in milk when the lactation period was longer, as well as a lower average size obtained with dynamic light scattering (DLS) analysis (Fig. 1B). By TEM it was observed that exosomes have a round shape with a diameter of 140 nm (Fig. 1C).

The expression of exosome markers such as ALIX, CD9 and CD63 was analyzed by western-blot (Fig. 1D). Finally, the HBM exosome uptake capacity was explored in several tumor cell lines of different origin: HeLa (cervix), B16F10 (murine melanoma), and 8505C (thyroid) (Fig. 1E). The exosomes labeled with a lipophilic carbocyanin green fluorescence probe (PKH67). The results showed that HBM exosomes are efficiently integrated into different types of tumor cells regardless of their cellular origin.

**Example 2. Characterization of hNIS expression in HBM exosomes.**

The role of the expression of NIS in the lactating mammary gland is the accumulation of iodine in breast milk, so that it passes to the nursing baby and he/she has iodine for the synthesis of their own thyroid hormones. It has also been defined that this iodine could play a protective role against infections. NIS is expressed in the mammary gland cells only during the period of breastfeeding. Here, the goal was to determine whether exosomes could be obtained from human breast milk during the lactation period, and to analyze whether those exosomes had endogenous expression of NIS. By means of western-blot (Fig. 2) it was seen that the exosomes extracted from HBM effectively express NIS regardless of the time of lactation that the donors had and their expression did not decay over time.

**Example 3. Functional characterization of NIS in human breast milk exosomes.**

The critical point for using NIS as a theragnostic tool is its ability to accumulate radioactive isotopes. To determine the functionality of the endogenous expression of NIS, *in vitro* radioactive iodide accumulation experiments were performed (Fig. 3).

The most interesting of the results is that HeLa tumor cells incorporated exosomes isolated from human breast milk, and accumulated iodide at levels similar to those obtained in the positive control (Fig. 3). Control are HeLa cells, which do not endogenously express NIS, infected with 5 and 50 pfu of an adenovirus that expressed NIS (Ad-CMV-hNIS). These results indicate that hNIS present in exosomes has been transferred to the target cell, being incorporated into the plasma membrane of this cell, and that it is functional. These results confirm that breast milk exosomes express functional NIS and suggest that they could be used as therapeutic vehicles.

**Example 4. Biodistribution of HBM exosomes in animal tumor models by Fluorescence Imaging.**

The next objective was to determine the *in vivo* biodistribution of breast milk exosomes in non-tumor tissues and their possible tropism towards tumors. For this it is used non-invasive imaging techniques. The first technique used was fluorescence imaging (FLI). Nude mice injected subcutaneously with HeLa tumor cells were used to assess *in vivo* biodistribution. Once that the subcutaneous tumor reached an approximate size of 0.5 cm$^2$, the intravenous administration of 20 μg of exosomes isolated from breast milk labeled with the fluorescent probe PKH67 (Sigma-Aldrich). The signal emitted by the probe in the mice was detected with an IVIS Illumina II 24 and 48 hours after injection (FIG. 4). Mice administered PBS and/or mice injected with the same amount of fluorescent probe were used as experimental control.

Subsequently, the animals were sacrificed, the different organs separated and the signal of each one of them was quantified *ex vivo* with the Xenogen-IVIS software. It was found that the signal from breast milk exosomes accumulates mostly in tumors (Fig. 4). Fluorescent signals were observed in other tissues, although partly is due to retention of the probe itself in those tissues as can be seen in the controls (Fig. 4). Treatment with 40 μg of exosomes did not result in a significant enhancement of accumulation in tumors, but a significant increase in signal in other non-specific tissues (data not shown). Similar results were obtained using EVs from HBM instead of exosomes (data not shown), but exosomes were chosen to perform the following experiments due to the greater purity and homogeneity of the samples.

**Example 5. Analysis of the biodistribution of HBM exosomes *in vivo* by SPECT-CT.**

Using the same *in vivo* model of subcutaneous tumors produced by HeLa cells as in the previous sections, and 7 days after the injection of the breast milk exosomes, the animals were injected intraperitoneally with 18.5 MBq of $^{99m}$TcO4-. The distribution of the radioisotope and, therefore, of the presence of NIS *in vivo,* was obtained using SPECT-CT (Fig. 5). Accumulation of the radiotracer can be observed in the tumors of the animals treated with HBM exosomes (FIG. 5B), and not in the controls (FIG. 5A).

It can be seen that this accumulation is not as high as it occurs in tissues such as the stomach or bladder. However, in the latter, the tracer, unlike what happens in the tumor, is passing through and does not accumulate. The signal was maintained for at least 14 days in the tumor. Therefore, the HBM exosomes analyzed have natural tropism for tumors and endogenous hNIS is transferred to the tumor, demonstrating that it is functional.

## Example 6. Therapeutic potential of breast milk exosomes.

In the previous section, it can be seen that the amount of radioisotope accumulated by tumors is apparently not very high. So, the next step was to explore if that expression is enough for an adequate therapy. For this, the *in vivo* model of the previous sections was used again. The therapeutic effect of radioiodine (RAI) therapy was tested when exosomes isolated from human breast milk were injected into tumor-bearing animals (Fig. 6). 7 days after the implantation of the tumor cells, the animals were injected intravenously with 20 $\mu$g maternal milk exosomes. PBS was injected into the controls. After 4 days of this injection, the therapeutic treatment was carried out with RAI or with PBS as a control. The approximate pre-RAI tumor size was 200 mm$^3$. In the subgroups where exosomes were injected and treated with RAI, the tumor grew more than twice, but in the rest of the groups, both controls and those not treated with RAI, the tumor grew more than 5-7.5 times. These results revealed the great effectiveness of RAI as *in vivo* antitumor therapy when combined with exosomes from breast milk.

## Example 7. Theragnostic potential of human breast milk exosomes in metastases.

To determine whether HBM exosomes could be used as theragnostic agents in the clinic for both early diagnosis of metastases and possible combined treatment with $^{131}$I, an *in vivo* multinodular model was used by implanting tumors intraperitoneally with SKOV3-LUC. Once after the tumors were observed by bioluminescence, 20 $\mu$g of HBM exosomes labeled with a fluorescent probe were administered. Subsequently, images of the tumors by bioluminescence (Fig. 7A) and of the accumulation of exosomes by fluorescence (Fig. 7B) were acquired. It is clearly seen that the latter are located in tumors. Finally, it was analyzed whether the expression of NIS from HBM exosomes accumulated in tumors and metastases was functional by SPECT/CT. A clear accumulation of the radioisotope $^{99m}$TcO4- was observed in scattered tumor nodules in the peritoneum (Fig. 7C and D). This confirms the diagnostic (and possibly therapeutic) power of HBM exosomes in metastases.

## Example 8. Summary of the results.

The endogenous expression of hNIS in breast milk exosomes has been identified and characterized (Figure 2). Furthermore, it has been shown:

- That breast milk exosomes migrate to tumor cells by non-radioactive imaging (Figure 4) and using NIS-accumulative radioisotope imaging (Figure 5).
- That transfer of hNIS from milk exosomes to the tumor allows accumulation of radioisotopes accumulative by NIS in the tumor (Figure 5 and 6).

- That this accumulation of radioisotopes transportable by NIS with high energy levels (in this case $^{131}$I) leads to a very significant reduction in tumor growth (Figure 6).
- That milk exosomes also migrate towards tumor metastases (Figure 7) and that they are capable of incorporating radioisotopes transportable by NIS (Figure 7).

## Example 9. Materials and methods.

### 9.1. Cell cultures and treatments

#### Cell lines

HeLa cells: The HeLa cell line (ATCC CCL-2) is an immortalized adherent cell line derived from human cervical adenocarcinoma. This cell line was cultured and maintained in DMEM 4.5 g/l glucose, supplemented with 10% fetal bovine serum, 5% antibiotics/antimycotics, and 200mM L-glutamine.

8505C cells: 8505C cells came from an undifferentiated human thyroid carcinoma. 8505C were cultured and maintained in RPMI-1640 medium (Lonza) supplemented with 10% fetal bovine serum, 5% antifungal antibiotics, and 200mM L-glutamine.

B16-F10 cells: B16-F10 cells came from a mouse melanoma cell line, characterized by very rapid growth and being highly invasive or metastatic. They were grown and maintained in DMEM medium 4.5 g/l glucose, supplemented with 10% fetal bovine serum, 5% antifungal antibiotics and 200 mM L-glutamine, as indicated above.

#### Maintenance of cells in culture

The cell lines used were kept inside an incubator at 5% $CO_2$ and at 37°C, in different culture plates with a surface area of 60, 100 and/or 150 cm$^2$, in culture cell flasks with a surface area of 25, 75 and/or 150 cm$^2$ or in multiwell plates with 6, 12, 24 and/or 96 wells depending on the objective of the experiment. Cells were grown under semi-confluent conditions and were trypsinized every 3-4 days.

#### Cellular passages

When the cells reached confluency, it was necessary to transfer them to another plate to allow them to continue growing. To do this, the supernatant was aspirated and the plate was washed with PBS-EDTA 1X (Lonza), eliminating any remaining serum that could inactivate trypsin, trypsin 0.05% 1X (Lonza) was added to the cells and allowed to act for 3-5 minutes. When the cells were detached, complete medium was added to inactivate trypsin, all the medium was collected by adding it in a falcon and centrifuged at 1000 rpm for 5 min to precipitate the cells. Subsequently, the supernatant was aspirated, the pellet was resuspended in fresh medium and the desired number of cells was seeded in a new plate. To determine the cell density, deposited 10 μl of cell suspension in a Neubauer chamber (BRAND®), the cells present in it were counted using a Leica DMi1 phase contrast microscope (Leica Microsystmes) and the cell concentration was estimated.

Freeze and thaw

After trypsinizing and centrifuging the cells at 1,000 rpm, the pellet was resuspended in freezing medium consisting of 90% fetal bovine serum or FBS (Lonza) and 10% dimethyl sulfoxide (DMSO) (Sigma-Aldrich) and introduced in cryovials. The decrease in temperature during the freezing process was carried out in a gradually using freezing containers (Nalgene® Mr. Frosty® Cryo 1°C Freezing Containers, ThermoScientific) and placing them in the -80°C freezer. After 48 hours, the cryovials can be transferred to the liquid nitrogen container where they will be stored until their use is required.

For thawing, cryovials removed from liquid nitrogen were rapidly placed in a 37°C bath. When the freezing medium was practically thawed, it was diluted in complete medium and centrifuged at 1000 rpm. The cell pellet was resuspended in complete medium and seeded in a culture plate or flask, changing the medium 24 hours after thawing.

Adenoviral Vectors and Infections

The adenoviral vector used was Ad-hNIS (also known as Ad10), built on the basis of adenovirus serotype 5 and the human NIS gene preceded by the promoter of cytomegalovirus (CMV) using the AdEasy system. The Ad-hNIS was designed and provided by Dr. Georges Vassaux (INSERM, Nice, France). The number of infective viral particles per cell (pfu/cell) present in the culture medium was expressed as multiplicity of infection (MOI). To carry out the adenoviral infection, the cells were seeded at the desired cell concentration and kept in culture for 24 hours until the confluency was 70%. Ad-CMV-hNIS was then diluted in serum-free medium at different multiplicities of infection (5 and 50 pfu), added to the cells and gently rocked the plate to ensure that it was well distributed throughout the culture surface. The volume used to infect the cells was ¼ of the total final volume in which the cells are incubated in the plate or well, which covers the entire surface of the culture and maximizes the number of interactions between the viruses and the cells. The cells were incubated with the infection medium at 37°C for 45 min. Subsequently, complete fresh medium was added to complete the total final volume and they were incubated at 37°C until they were used.

The volume of virus used was calculated using the following formula: Virus volume (μl)= (MOI x number of cells)/(Viral titer)

Being MOI the multiplicity of viral infection, that is, the number of infected particles per cell (pfu/cell) to which the infection was made and the viral titer being the number of viral particles in 1 ml.

Exosome Treatments

For the *in vitro* exosome uptake and internalization experiments, different HeLa, B16-F10 and 8505C tumor cell lines were used. The cells were seeded at the desired density, depending on the culture surface used, in complete DMEM medium and incubated for 16-24 hours at 37°C. After that time, they were washed twice with PBS 1X and incubated with different amounts of the exosomes of interest or PBS, in the case of controls, diluted in a minimum volume of complete DMEM medium without exosomes for 45 minutes at 37°C. The minimum volume used allowed to cover the entire cultivation surface and corresponds with ¼ of the total final volume in which the cells are incubated in the wells or culture plates used. After that time, 2 washes were performed. Complete fresh medium without exosomes was added and incubated for 48 hours before performing the experiments of interest. Different exosome administration protocols were followed, adding a single dose of 20 μg or 40 μg of exosomes or adding two serial doses of 20 μg separated by a time interval of 24 hours.

**9.2. Isolation and quantification of exosomes**

Isolation of exosomes from HBM

The milk from the various donors was frozen until extraction. For extraction, the samples are thawed at 4°C and the fat is removed by double centrifugation at 3000g for 10 min at room temperature. To 50 ml of the supernatant, 500 μl of rennet was added and incubated for 20 minutes at 37°C. Subsequently, it was centrifuged at 4°C for 30 minutes at 5000g to remove casein and other products of proteolysis.

The supernatant followed a protocol similar to that of the rest of the exosome extractions, in which a first centrifugation of 30 minutes at 13,000g at 4°C was carried out, in which the EVs remain in the pellet. After ultracentrifugation at 100,000 g and 4°C for 60 minutes, followed by two other washes with PBS at 135,000 g for 90 minutes, the exosomes resuspended in HEPES (Beckman Coulter TL-100 Centrifuge) were obtained.

Quantification of exosomes

The quantification of isolated extracellular vesicles, exosomes and microvesicles, was estimated by determining the total protein concentration using the bicinchonic acid or BCA-based colorimetric method (Pierce, Thermo Scientific). For this purpose, a calibration curve or standard straight line was established using concentrations known levels of bovine serum albumin (BSA) ranging from 0-8 $\mu g/\mu l$. The concentration of the samples was determined in triplicate. For this, a 96-well plate was used, where the corresponding samples with the values of the standard line and/or with the exosome samples were deposited and 200 $\mu l$ of the reagent was added, following the manufacturer's instructions. Subsequently, it was incubated in darkness at 37°C for 30 minutes and the absorbance measurement of the plate at 562 nm was performed using a TECAN ® (SPARK) equipment. The concentration of exosomes was calculated by extrapolating the absorbance values in the previously established standard line.

### 9.3. Characterization of exosomes

The morphological analysis of the exosomes was performed by transmission electron microscopy using a T20-FEI transmission microscope (Tecnai, Thermoionic transmission electron microscope).

Determination of the size and concentration of the isolated extracellular vesicles were performed through nanoparticle tracking analysis (NTA). It is a technique that uses light scattering and Brownian motion to obtain the size distribution and concentration of particles in suspension. To do so, the isolated exosomes were diluted in PBS and analyzed using a NanoSight NS500 (Malvern Instruments GmbH) equipped with four lasers at a wavelength of 405 nm, 488 nm, 532 nm and 638 nm and a high-sensitivity sCMOS camera. Samples were manually entered and three 30-second videos were recorded at a frame rate of 25 frames per second. NTA 3.0 software was used to monitor individual particles at camera level 10, with the algorithm of Finite Track Length Adjustment (FTLA) set to an automatic minimum length and conservative blur detection threshold.

The zeta potential or electrokinetic potential is an average of the magnitude of the electrostatic interactions, repulsion or attraction, of the particles. Its measurement provides information on the stability of the particles and their state of aggregation. To determine the zeta potential, dynamic light scattering (DLS) was measured. The isolated exosomes were diluted in 1X PBS and placed in a photomultiplier tube placed at 90° to the incident light beam for analysis. The team A Zetasizer NanoSERIES (Malvern Instruments GmbH) equipped with a 20 mW helium/neon laser at a wavelength of 633 nm was used.

### 9.4. Protein expression analysis

Western-blot immunodetection

This technique was used to analyze the expression of the different proteins of interest. Following quantification with the BCA method as described above, exosomes were directly resuspended in Laemmli loading buffer, containing DTT (a reducing agent) and heated at 37°C for 30 min causing protein denaturation before loading into the discontinuous 5 9% polyacrylamide gel. The molecular weight marker used was Prestained Protein Ladder-Mid-range molecular weight (Abcam). The top gel or concentrator gel contained a constant low percentage of acrylamide for each gel and a pH of 6.8. While the lower gel or separator was characterized by having a higher percentage of acrylamide, which will vary depending on the size of the protein to be separated, and a pH of 8.8. Electrophoresis was run for 20-30 minutes at 40V to facilitate the entry of the samples into the gel and later at 100-120V, allowing the samples to migrate until the bromophenol blue front present in the loading buffer came out of the gel. To perform the electrophoresis, a vertical Mini-PROTEAN system (Bio Rad) was used. Subsequently, the transfer was performed at 100 V for 1.5 hours in transfer buffer, the membrane was blocked for 1 hour with blocking solution, Tween-TBS (T-TBS) buffer with 5% (w/v) skimmed milk powder and incubated with the desired antibody at its optimal concentration in blocking solution overnight at 4°C. The next day, the membrane was washed with T-TBS buffer for 10 minutes and the membrane was incubated with the secondary antibody associated with horseradish peroxidase (HRP) for 1 hour at room temperature. Finally, the membrane was washed 2 times with T-TBS for 10 minutes and once more with TBS for another 10 minutes, and the membrane was developed using an ECL chemiluminescent detection system (Amersham Pharmacia Biotech). The image capture system used was the Amersham Imager 680 (GE Healthcare Life Science). After detection of the antibody of interest, the PVDF membranes were incubated with 1X RestoreTM Western Blot Stripping Buffer removing solution under agitation for 15 minutes, the membrane was blocked with blocking solution for 30 minutes and the membranes continued to incubate. with the antibodies following the protocol described above.

Immunodetection of human NIS was performed using mouse-generated anti-NIS monoclonal antibody TE2A3 (1:500) and HRP-linked anti-rabbit IgG (1:2000; Chemicon International) was used as the secondary antibody.

Immunofluorescence and confocal microscopy

For the *in vitro* exosome uptake and internalization experiments, different HeLa, B16-F10 and 8505C tumor cell lines were used. 2 x $10^4$ cells were seeded on 12 mm diameter coverslips in 24-well plates for 16-24 hours in complete DMEM medium, before being washed twice with PBS and incubated with different amounts (20 $\mu g$, 40 $\mu g$ or 20+20 $\mu g$) of the exosomes in a minimum volume of complete DMEM medium without exosomes for 45 minutes at 37°C. The minimum volume used allows to cover the entire culture surface and corresponds to ¼ of the total final volume in which the cells are incubated in the well. Complete fresh medium without exosomes was then added and incubated 48 hours. Subsequently, the cells on the coverslips were fixed and the immunofluorescence protocol was continued, for which they were incubated with the primary anti-NIS TE2A3 antibody diluted in PBS-BSA at room temperature in a humid chamber for 1 hour, washed with PBS three times, and incubated with the secondary antibody for 1 hour at room temperature. Finally, staining was analyzed immunofluorescent using a Nikon ECLIPSE TS100 inverted microscope (Nikon Instruments Europeam BV; Amsterdam, The Netherlands) connected to a UV light lamp.

Iodine uptake assay

The Iodine uptake assays allow analysis of NIS functionality. For the experiments on the *in vitro* study of the functionality of NIS present in exosomes, the HeLa cell line was used. After seeding the cells in 12-well plates, they were incubated for 24 hours at 37°C. The cells were then washed a times with PBS 1X and incubated in a minimum volume of complete DMEM medium without exosomes to which a certain amount of exosomes was added for 45 minutes at 37°C. After that time, complete fresh medium without exosomes was added and incubated for 48 hours. Subsequently, the uptake assay protocol was continued.

The previously mentioned uptake assay consisted of incubation for 1 hour at 37°C with PBS supplemented with 20 $\mu$M IK and 100 $\mu$Ci 125 I/$\mu$mol I-. As an additional control to determine that iodide transport is specifically due to NIS, the cells were incubated with the previous solution and with 80 $\mu$M NaClO$^4$, a compound that inhibits competitively NIS-dependent iodine ion flux. when the transport of iodine reached equilibrium, the radioactive medium was aspirated, the wells washed twice and incubated with cold absolute ethanol at 4°C for 20 minutes, to allow the iodine accumulated in the cells to escape into the medium. Radioactivity was quantified in a gamma scintillation counter (Wallac Wizard 3' 1480 Autimatic Gamma Counter, Perkin Elmer). To normalize the values obtained by the number of cells in each well, the DNA was precipitated with trichloroacetic acid (Sigma-Aldrich) and quantified with the diphenylamine method. Iodine uptake was expressed as pmol I-/$\mu$g DNA in the well. The results are the average of at least 3 different experiments, performing in each experiment triplicates or quadruplicates of each one of the conditions analyzed.

9.5. *In vivo* methods

Induction of the tumor model *in vivo*

To carry out the *in vivo* experimentation, female BALB/c nu/nu mice aged between 6-8 weeks from Harlan-Ibérica (Harlan-Ibérica, Barcelona, Spain) were used. The animals were housed and kept in individually ventilated cages (IVC) under sterile conditions and the food and water provided were previously sterilized. Before performing any experimental procedures, the mice were given four days to acclimatize. After that time, 2 x $10^6$ HeLa cells resuspended in 100 $\mu$l of PBS were injected subcutaneously (sc) to generate subcutaneous tumors in the flank of the animals.

Tumor growth was monitored using calipers to measure the width and length of the tumor every 2 days. The following formula was used to calculate the tumor surface:

$$V=4/3 * \pi * a^2 * b$$

where a and b are the wide and long radio, respectively.

When the tumors reached the appropriate size, around 0.5 cm$^2$ in area, the mice were randomly separated into the different experimental groups and the experimentation proceeded. In order to establish the natural tropism in live exosomes, different doses of exosomes (20 $\mu$g, 40 $\mu$g or 20+20 $\mu$g) resuspended in 100 $\mu$l of PBS using different routes of administration such as intravenous (iv) or intraperitoneal (ip) to each mouse. While PBS was administered exclusively as a control.

Study of tropism using molecular imaging techniques

Different non-invasive imaging techniques were used, such as fluorescence imaging (FLI) and SPECT/CT, which allowed *in vivo* monitoring and localization of the administered exosomes thanks to the labeling carried out with fluorescent probes and by the endogenous expression of NIS.

To carry out the image by fluorescence or FLI (for its acronym in English, Fluorescence Imaging) it was necessary to label the exosomes with a fluorescent probe. Therefore, the fluorescent probe PKH67 Fluorescent Cell Linker Kit was used, which emits fluorescence in the spectrum of green or Claret that emits in the Far Red range (Sigma-Aldrich). Labeling with the fluorescent probe was performed following the manufacturer's instructions. *In vivo* scans were performed at 2, 24, 48, 72 hours and 7 days using an IVIS Lumina II (Xenogen Corporation, USA).

To obtain the fluorescent images, the animals were anesthetized in hermetically sealed induction boxes with a mixture of oxygen (1 I/minute) and 3-4% isoflurane (ISOVET). When the mice were completely anesthetized, they were transferred inside the IVIS Lumina II scanner previously warmed to 37°C and placed on individual mouthpieces to maintain anesthesia, maintaining the isoflurane administration at 2%. Acquisitions were performed using specific excitation and emission filters for each zone, being necessary to take a first capture to eliminate the fluorescence inside the scanner. Images were taken with an exposure time of 30 minutes, small binning and an F-Stop of 2. For animals or samples with high intensity, it was necessary to reduce the exposure time to 1 minute or even 30 seconds. Two scans of each animal were made, in a dorsal position and in a ventral position. After the acquisitions, the animals were returned to their IVC cages where they recovered from anesthesia. After the various times, the animals were euthanized by cervical dislocation, organs processed and scanned *exlive.* Additionally, they were analyzed using different techniques, such as bioluminescence, immunofluorescence or immunodetection, in order to analyze the biodistribution of exosomes. Data were acquired and analyzed with Living Image 4.0 software (Xenogen Corporation). The quantification of the signal fluorescence was performed by delimiting the region of interest or ROI (for its acronym in English, Region Of Interest) of each animal or organ during each of the scans.

SPECT/CT is a non-invasive molecular imaging technology that combines two imaging systems, Single Photon Emission Computed Tomography (SPECT) and Computerized Tomography (CT), allowing to obtain three-dimensional (3D) images of the biodistribution of the administered photon-emitting radioisotope (gamma emitter) that will be combined with the anatomical images obtained by applying X-rays. To achieve these images, hNIS was used as a visualization tool and a photon-emitting radioisotope such as $^{99m}TcO4$, enabling the identification of those areas where the expression of NIS is located thanks to the accumulation of $^{99m}TcO4$- in them. Image acquisition was performed using a gamma camera that rotates around the study animal, obtaining two-dimensional (2D) projections or images in the sagittal, coronal, and transaxial planes. This is because the camera is used with a full 360-degree rotation. to optimize the reconstruction of the images obtained. The rotation is done in defined positions, which generally vary between 3 and 6 degrees. Subsequently, the 2D images or projections are processed and by applying mathematical algorithms the 3D images or projections are reconstructed, which are fused with the CT images, increasing the spatial resolution of the study and increasing the sensitivity of the resolution.

The performance of these experiments was based on the use of endogenous expression of hNIS in milk exosomes as a therapeutic tool. For this, it was necessary to inject milk exosomes into xenograft tumor models in mice following the protocol described above. Prior to the administration of the radioisotope, the animals received a 7-day treatment of L-tyrosine (T4) (Sigma-Aldrich) in water at a dose of 5 mg/l in order to block the expression of NIS in the thyroid. of the animals. *In vivo* scans were performed 7, 10, and 15 days after exosome injection using a Brucker Albira II SPECT/CT scanner (Bruker Medical GmbH). To do so, the animals received the administration intraperitoneal 18.5 MBq of $^{99m}TcO4$- resuspended in 200 $\mu$l of PBS. After 5 minutes, the animals were anesthetized and placed inside the Albira II SPECT/CT on a heated bed at 37°C and a probe was used to monitor their vital signs. SPECT and CT of the body were performed on the whole of the animal, taking acquisitions every 100 seconds. The reconstruction of the images to obtain the 3D image was performed using the MEDISO software (MEDISO Medical Imaging System) and the fusion of the SPECT and CT images was performed using the PMDO software (Biomedical Image Quantification, PMOD Technologies LLC). The calculation of the accumulation of the radioisotope 99mTcO4- was carried out using the *in vivo* software from Scope (Medical Image System).

Study of the therapeutic potential of the administration of $^{131}I$

The study of the therapeutic potential of the endogenous expression of hNIS in exosomes of HBM was performed by administering $^{131}I$, a radioiodine isotope routinely used in hospital radiotherapy treatments. In the experiments related to the therapeutic potential of the expression, the animals received an intravenous injection of exosomes and after 7 days, 2 mCi of ($^{131}I$) NaI resuspended in 200 $\mu$l of PBS were administered intraperitoneally. Prior to the injection of said radioiodine isotope, the animals received a treatment of L-tyrosine (T4) in the water at a concentration of 5mg/l to minimize thyroid damage due to radioisotope administration. To analyze the therapeutic effect, the tumor size was monitored every 2 days, during the days after the administration of the radioisotope, measuring the length and width of the tumors. The monitoring of the animals was carried out for 42 days. The results are the average of at least 2 different experiments, performing triplicates or quadruplicates of each of the conditions analyzed in each experiment.

**Claims**

1. Composition comprising:

   (i) extracellular vesicles, preferably exosomes, secreted by cells expressing the NIS protein, wherein said extracellular vesicles express the NIS protein, and
   (ii) (ii) at least one radioactive isotope,

   wherein the extracellular vesicles, preferably exosomes, secreted by cells expressing NIS protein have been isolated from milk.

2. Composition according to claim 1, wherein the radioactive isotope is selected from the list consisting of:$^{131}I$,$^{123}I$,$^{124}I$,$^{125}I$,$^{211}At$,$^{99m}TcO4$-,$^{188}ReO4$-, and$^{18}F$-BF4-, preferably wherein the radioactive isotope is $^{131}I$ or $^{99m}TcO4$-.

3. Composition according to claim 2, wherein the radioactive isotope is $^{131}I$.

**4.** Composition according to any one of claims 1 to 3, wherein the NIS protein is human.

**5.** Composition according to any one of claims 1 to 4, wherein the milk is breast milk from a female, preferably human, who is in the period of lactation.

**6.** Composition according to any one of claims 1 to 5, which is a pharmaceutical composition preferably formulated for intravenous or intraperitoneal administration.

**7.** Composition according to any one of claims 1 to 6 for use as a medicine.

**8.** Composition according to claim 7, for use in the treatment and/or prevention of neoplasms.

**9.** Composition for use according to claim 8, wherein said composition comprises the radioactive isotope $^{131}$I.

**10.** Composition for use according to any of claims 8 or 9, wherein the neoplasm is a solid or hematological tumor, preferably wherein the solid tumor is a cancer, more preferably wherein the cancer is selected from the list consisting of: melanoma, multiple myeloma, leukemia, lymphoma, ovarian cancer, cervical cancer, lung cancer, liver cancer, breast cancer, pancreas cancer, gastric cancer, colorectal cancer, head and neck cancer, mouth cancer, stomach cancer, brain tumors and thyroid cancer.

**11.** Use of the composition according to any one of claims 1 to 6 as an imaging agent for the visualization of neoplasms in a subject.

**12.** Use of the composition according to claim 11, wherein said composition comprises a radioactive isotope selected from the list consisting of: $^{123}$I,$^{124}$I, $^{125}$I, $^{211}$At, $^{99m}$TcO4-, $^{188}$ReO4-, and $^{18}$F-BF4-, preferably $^{124}$I and $^{99m}$TcO4-.

**13.** Use of the composition according to any of claims 11 or 12, wherein the neoplasm is a solid or hematological tumor, preferably wherein the solid tumor is a cancer.

**14.** Use of the composition according to claim 13, wherein the cancer is selected from the list consisting of: melanoma, multiple myeloma, leukemia, lymphoma, ovarian cancer, cervical cancer, lung cancer, liver cancer, breast cancer, pancreas cancer, gastric cancer, colorectal cancer, head and neck cancer, mouth cancer, stomach cancer, brain tumors and thyroid cancer.

**15.** Use of the composition according to any one of claims 11 to 14, wherein the visualization of neoplasms is performed using the PET or SPECT technique.

**Patentansprüche**

**1.** Zusammensetzung, umfassend:

(i) extrazelluläre Vesikel, vorzugsweise Exosomen, die von Zellen sekretiert werden, die das NIS-Protein exprimieren, wobei die extrazellulären Vesikel das NIS-Protein exprimieren, und
(ii) (ii) mindestens ein radioaktives Isotop,

wobei die extrazellulären Vesikel, vorzugsweise Exosomen, die von Zellen sekretiert werden, die das NIS-Protein exprimieren, aus Milch isoliert wurden.

**2.** Zusammensetzung nach Anspruch 1, wobei das radioaktive Isotop aus der Liste ausgewählt ist, bestehend aus: $^{131}$I, $^{123}$I, $^{124}$I, $^{125}$I, $^{211}$At, $^{99m}$TcO4-, $^{188}$ReO4-, und $^{18}$F-BF4-, vorzugsweise wobei das radioaktive Isotop $^{131}$I oder $^{99m}$TcO4- ist.

**3.** Zusammensetzung nach Anspruch 2, wobei das radioaktive Isotop $^{131}$I ist.

**4.** Zusammensetzung nach einem der Ansprüche 1 bis 3, wobei das NIS-Protein menschlich ist.

**5.** Zusammensetzung nach einem der Ansprüche 1 bis 4, wobei die Milch Muttermilch von einem weiblichen, vorzugsweise menschlichen, Individuum ist, das sich in der Stillphase befindet.

**6.** Zusammensetzung nach einem der Ansprüche 1 bis 5, die eine pharmazeutische Zusammensetzung ist, die vorzugsweise zur intravenösen oder intraperitonealen Verabreichung formuliert ist.

**7.** Zusammensetzung nach einem der Ansprüche 1 bis 6 zur Verwendung als Arzneimittel.

**8.** Zusammensetzung nach Anspruch 7 zur Verwendung bei der Behandlung und/oder Vorbeugung von Neoplasmen.

**9.** Zusammensetzung zur Verwendung nach Anspruch 8, wobei die Zusammensetzung das radioaktive Isotop $^{131}$I umfasst.

**10.** Zusammensetzung zur Verwendung nach einem der Ansprüche 8 oder 9, wobei das Neoplasma ein solider oder hämatologischer Tumor ist, vorzugsweise wobei der solide Tumor ein Krebs ist, noch bevorzugter wobei der Krebs aus der Liste ausgewählt ist, bestehend aus: Melanom, multiplem Myelom, Leukämie, Lymphom, Eierstockkrebs, Gebärmutterhalskrebs, Lungenkrebs, Leberkrebs, Brustkrebs, Bauchspeicheldrüsenkrebs, Magenkrebs, Darmkrebs, Kopf- und Halskrebs, Mundkrebs, Magenkrebs, Hirntumoren und Schilddrüsenkrebs.

11. Verwendung der Zusammensetzung nach einem der Ansprüche 1 bis 6 als Bildgebungsmittel zur Visualisierung von Neoplasmen in einem Patienten.

12. Verwendung der Zusammensetzung nach Anspruch 11, wobei die Zusammensetzung ein radioaktives Isotop umfasst, das aus der Liste ausgewählt ist, bestehend aus: $^{123}$I, $^{124}$I, $^{125}$I, $^{211}$At, $^{99m}$TcO4-, $^{188}$ReO4-, und $^{18}$F-BF4-, vorzugsweise $^{124}$I und $^{99m}$TcO4-.

13. Verwendung der Zusammensetzung nach einem der Ansprüche 11 oder 12, wobei das Neoplasma ein solider oder hämatologischer Tumor ist, vorzugsweise wobei der solide Tumor ein Krebs ist.

14. Verwendung der Zusammensetzung nach Anspruch 13, wobei der Krebs aus der Liste ausgewählt ist, bestehend aus: Melanom, multiplem Myelom, Leukämie, Lymphom, Eierstockkrebs, Gebärmutterhalskrebs, Lungenkrebs, Leberkrebs, Brustkrebs, Bauchspeicheldrüsenkrebs, Magenkrebs, Darmkrebs, Kopf- und Halskrebs, Mundkrebs, Magenkrebs, Hirntumoren und Schilddrüsenkrebs.

15. Verwendung der Zusammensetzung nach einem der Ansprüche 11 bis 14, wobei die Visualisierung von Neoplasmen unter Verwendung der PET- oder SPECT-Technik durchgeführt wird.

**Revendications**

1. Composition comprenant :

(i) des vésicules extracellulaires, de préférence des exosomes, sécrétées par des cellules exprimant la protéine NIS, dans laquelle lesdites vésicules extracellulaires expriment la protéine NIS, et
(ii) (ii) au moins un isotope radioactif,

dans laquelle les vésicules extracellulaires, de préférence des exosomes, sécrétées par des cellules exprimant la protéine NIS ont été isolées du lait.

2. Composition selon la revendication 1, dans laquelle l'isotope radioactif est choisi dans la liste constituée par : $^{131}$I, $^{123}$I, $^{124}$I, $^{125}$I, $^{211}$At, $^{99m}$TcO4-, $^{188}$ReO4-, et $^{18}$F-BF4-, de préférence dans laquelle l'isotope

radioactif est $^{131}$I ou $^{99m}$TcO4-.

3. Composition selon la revendication 2, dans laquelle l'isotope radioactif est $^{131}$I.

4. Composition selon l'une quelconque des revendications 1 à 3, dans laquelle la protéine NIS est humaine.

5. Composition selon l'une quelconque des revendications 1 à 4, dans laquelle le lait est du lait maternel provenant d'une femelle, de préférence humaine, qui est en période de lactation.

6. Composition selon l'une quelconque des revendications 1 à 5, qui est une composition pharmaceutique de préférence formulée pour une administration intraveineuse ou intrapéritonéale.

7. Composition selon l'une quelconque des revendications 1 à 6 destinée à être utilisée comme médicament.

8. Composition selon la revendication 7, destinée à être utilisée dans le traitement et/ou la prévention de néoplasmes.

9. Composition destinée à être utilisée selon la revendication 8, dans laquelle ladite composition comprend l'isotope radioactif $^{131}$I.

10. Composition destinée à être utilisée selon l'une quelconque des revendications 8 ou 9, dans laquelle le néoplasme est une tumeur solide ou hématologique, de préférence dans laquelle la tumeur solide est un cancer, de préférence dans laquelle le cancer est choisi dans la liste constituée par : le mélanome, le myélome multiple, la leucémie, le lymphome, le cancer de l'ovaire, le cancer du col de l'utérus, le cancer du poumon, le cancer du foie, le cancer du sein, le cancer du pancréas, le cancer gastrique, le cancer colorectal, le cancer de la tête et du cou, le cancer de la bouche, le cancer de l'estomac, les tumeurs cérébrales et le cancer de la thyroïde.

11. Utilisation de la composition selon l'une quelconque des revendications 1 à 6 comme agent d'imagerie pour la visualisation de néoplasmes chez un sujet.

12. Utilisation de la composition selon la revendication 11, dans laquelle ladite composition comprend un isotope radioactif choisi dans la liste constituée par : $^{123}$I, $^{124}$I, $^{125}$I, $^{211}$At, $^{99m}$TcO4-, $^{188}$ReO4-, et $^{18}$F-BF4-, de préférence $^{124}$I et $^{99m}$TcO4-.

13. Utilisation de la composition selon l'une quelconque des revendications 11 ou 12, dans laquelle le néoplasme est une tumeur solide ou hématologique, de préférence dans laquelle la tumeur solide est un cancer.

14. Utilisation de la composition selon la revendication 13, dans laquelle le cancer est choisi dans la liste constituée par : le mélanome, le myélome multiple, la leucémie, le lymphome, le cancer de l'ovaire, le cancer du col de l'utérus, le cancer du poumon, le cancer du foie, le cancer du sein, le cancer du pancréas, le cancer gastrique, le cancer colorectal, le cancer de la tête et du cou, le cancer de la bouche, le cancer de l'estomac, les tumeurs cérébrales et le cancer de la thyroïde.

15. Utilisation de la composition selon l'une quelconque des revendications 11 à 14, dans laquelle la visualisation de néoplasmes est réalisée à l'aide de la technique PET ou SPECT.

Fig. 1

## Fig. 1 (cont.)

## Fig. 2

**Fig. 3**

Fig. 4

**Fig. 5**

A CONTROL

B HBM Exosomes

Fig. 6

Fig. 7

A

B

C

D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- De la Vieja Antonio, Santisteban Pilar. *Endocrine-Related Cancer*, 2018, vol. 25 (4), R225-R245
- **CHUNG, JK ; GJ CHEON**. *Endocrinol Metab*, 2014, vol. 29 (3), 233-239
- **CRESPO-BARREDA A., et al.** *Journal of Extracellular Vesicles*, 2019, vol. 8 (1), 105-106
- **CRESPO-BARREDA A. et al.** *Human Gene Therapy*, 2016, vol. 27 (11), A91
- **CRESPO-BARREDA A. et al.** *Human Gene Therapy*, 2019, vol. 30 (11), A53
- **AGRAWAL ASHISH K. et al.** *Nanomedicine*, 2017, vol. 13 (5), 1627-1636
- **MUNAGALA RADHA et al.** *Cancer Letters*, 2016, vol. 371 (1), 48-61
- **TAZEBAY UYGAR H. et al.** *Nature Medicine*, 2000, vol. 6 (8), 871-878
- *Drug Dev Ind Pharm.*, March 2019, vol. 45 (3), 359-364
- *Reprint Biol.*, December 2017, vol. 17 (4), 341-348
- *J. Vet. Med. Sci.*, 2012, vol. 74 (11), 1523-1525